# FASCICULE DE BREVET EUROPEEN

(11) **EP 0 862 927 B1**
(45) Date de publication et mention de la délivrance du brevet: **08.09.2004**
(21) Numéro de dépôt: 98400540.5
(22) Date de dépôt: 06.03.1998
(51) Int. Cl.: A61N 1/365, A61N 1/368

(54) **Stimulateur cardiaque multisites pour le traitement des insuffisances cardiaques par stimulation**
Multi-Ort Herzschrittmacher zur Behandlung von Herzinsuffizienzen mit Reizimpulsen
Multi-site cardiac stimulator for the treatment of cardiac deficiencies by stimulation

(30) Priorité: 07.03.1997 FR 9702754
(43) Date de publication de la demande: 09.09.1998
(73) Titulaire: ELA MEDICAL (Société anonyme), 92541 Montrouge (FR)
(72) Inventeur: Cazeau, Serge, 75015 Paris (FR); Limousin, Marcel, 75014 Paris (FR); Ritter, Philippe, 92290 Chatenay Malabry (FR)
(74) Mandataire: Dupuis-Latour, Dominique

(56) Documents cités:
- EP-A- 0 522 693
- US-A- 5 139 020
- US-A- 5 174 289
- US-A- 5 584 868
- SCHALDACH M: "PEP-GESTEUERTER HERZSCHRITTMACHER" BIOMEDIZINISCHE TECHNIK, vol. 34, no. 7/8, juillet 1989, pages 177-184, XP000050247

## Description

La présente invention concerne les stimulateurs cardiaques (ou les circuits de stimulation d'un défibrillateur ou cardioverteur) dans leur utilisation pour le traitement de l'insuffisance cardiaque par stimulation.

En effet, outre le traitement des troubles du rythme cardiaque, différents auteurs (Hochleitner, Bekker, Cazeau) ont proposé de traiter par stimulation les troubles de contraction myocardique observés chez des patients en insuffisance cardiaque, ces troubles pouvant être spontanés ou induits par une stimulation traditionnelle.

En effet, on a observé que des patients présentant des troubles de classe NYHA 3 et 4 pouvaient voir leur condition améliorée par une stimulation appropriée. Il a été notamment proposé, dans une première expérience, d'utiliser un stimulateur double chambre conventionnel en programmant un délai atrio-ventriculaire court. Dans des études plus récentes, il a été proposé de stimuler les cavités gauche et droite dans des modèles triple chambre ou quadruple chambre.

Mais, dans tous les cas, il n'a jamais été clairement défini de critère d'implantation (hormis l'insuffisance cardiaque) ni d'explication sur les mécanismes impliqués dans la correction des troubles cardiaques. La programmation des différents paramètres de stimulation est donc, dans l'état actuel des connaissances, individualisée empiriquement pour chaque patient et par chaque praticien.

L'un des buts de la présente invention est de pallier ces limitations en proposant un stimulateur cardiaque susceptible d'optimiser les paramètres de stimulation de façon objective et déterministe (c'est-à-dire sans appréciation personnelle du praticien), automatique et adaptative au cours du temps en fonction de l'évolution, à court terme et à long terme, de l'état fonctionnel du patient.

Sur les patients appareillés, des examens échocardiographiques ont montré que le trouble cardiaque était influencé par l'intervalle de temps séparant la dépolarisation ou l'impulsion de stimulation ventriculaire d'une part, et le début de l'éjection ventriculaire d'autre part. Cet intervalle correspond en fait à l'ouverture des valvules sigmoïdes (valvule pulmonaire pour le ventricule droit et valvule aortique pour le ventricule gauche), indiquant le début de la phase de contraction systolique, phase qui succède à la phase de contraction isovolumétrique du ventricule correspondant, ou phase d'éjection.

On a également constaté que l'intervalle en question est influencé par le choix du site de stimulation, c'est-à-dire que, en d'autres termes, l'intervalle n'est pas le même selon le site choisi sur le ventricule.

Essentiellement, l'invention propose d'utiliser un stimulateur cardiaque du type "multisites" et de prévoir des moyens pour tester les différents sites possibles et choisir celui procurant l'intervalle dépolarisation-début d'ouverture le plus faible, et d'opérer la stimulation avec cette configuration.

Un stimulateur "multisites" est un stimulateur dans lequel des électrodes sont placées en une pluralité de sites respectifs distincts, avec au moins deux sites ventriculaires. Il peut s'agir d'un stimulateur de type "simple chambre" (double stimulation ventriculaire), triple chambre (stimulation atriale droite et double stimulation ventriculaire) ou même quadruple chambre (double stimulation atriale et double stimulation ventriculaire).

Plus précisément, le stimulateur de l'invention, qui est un stimulateur du type "multisites" précité, c'est-à-dire dans lequel des électrodes sont placées en une pluralité de sites respectifs distincts comprenant au moins deux sites ventriculaires, ces électrodes étant reliées à des bornes indépendantes du stimulateur de manière à permettre la détection d'un potentiel de dépolarisation ainsi que l'application d'une impulsion de stimulation, est caractérisé par : un capteur de contraction d'au moins l'un des ventricules, pour détecter un instant de début d'ouverture valvulaire ; des moyens de recherche de configuration optimale, pour commuter successivement et automatiquement les électrodes ventriculaires selon diverses configurations possibles, et pour détecter un instant de dépolarisation ou de stimulation sur l'électrode dans la configuration correspondante, déterminer l'intervalle temporel séparant, pour une configuration donnée, l'instant de dépolarisation ou de stimulation et l'instant de début d'ouverture valvulaire, et sélectionner la configuration procurant l'intervalle temporel le plus court ; et des moyens pour appliquer une stimulation sur l'électrode dans la configuration sélectionnée par les moyens de recherche.

Selon diverses caractéristiques avantageuses subsidiaires :
― il est prévu des moyens pour détecter des extrasystoles auriculaires et ventriculaires, et inhiber les moyens de recherche en cas de détection d'une extrasystole ;
― il est prévu des moyens pour comparer la valeur de l'intervalle temporel déterminée pour un cycle cardiaque donné à la valeur correspondante mémorisée pour un cycle antérieur, et mettre à nouveau en oeuvre les moyens de recherche en cas d'augmentation de ladite valeur ;
― il est prévu des moyens pour déterminer un état de repos physiologique du patient et mettre à nouveau en oeuvre les moyens de recherche lorsque l'état de repos est détecté ;
― les moyens de recherche sont mis en oeuvre à intervalles réguliers, ou régulièrement après comptage d'un nombre prédéterminé de cycles ;
― il est en outre prévu des moyens pour adapter les délais entre les sites de stimulation.

D'autres caractéristiques et avantages de l'invention apparaîtront à la lecture de la description d'un exemple de réalisation ci-dessous.

Le stimulateur de l'invention est une prothèse implantée (stimulateur, défibrillateur ou cardioverteur) de type multisites, dans lequel les différents paramètres, notamment la configuration des sites de stimulation, sont choisis de manière à optimiser l'efficacité du traitement de l'insuffisance cardiaque.

Le stimulateur de l'invention est un stimulateur de type en lui-même connu, c'est-à-dire une prothèse de stimulation ou de défibrillation cardiaque simple ou double chambre dotée :
― d'au moins deux bornes de détection/stimulation ventriculaires indépendantes,
― d'une ou plusieurs bornes de détection/stimulation atriales indépendantes, suivant le type d'appareil (double, triple ou quadruple chambre),
― d'un capteur de contraction d'au moins l'un des ventricules, typiquement du ventricule gauche.

Le capteur de contraction ventriculaire a pour objet de mesurer des variations soit du volume, soit du mouvement des fibres musculaires ventriculaires en début de systole, et/ou de détecter l'ouverture d'une ou des deux valvules sigmoïdes, de manière à déterminer l'instant d'ouverture de cette valvule. Cet instant correspond à la transition entre la phase de contraction systolique isovolumétrique du ventricule et la phase d'éjection vers l'aorte (pour le coeur gauche) ou l'artère pulmonaire (pour le coeur droit). Ce paramètre peut être déterminé par divers types de capteurs connus tels que capteur de mesure de l'impédance électrique du myocarde, capteur de mesure de la contractilité, capteur de mesure du volume ventriculaire par magnétométrie, capteur de détection d'ouverture de la valvule par transducteur à ultrasons, etc. Des capteurs montés sur une sonde de stimulation pour la détection de l'ouverture des valvules sont décrits dans le US-A-5 243 976 et, pour la mesure de la contractilité, dans le US-A-5 154 171.

On peut ainsi appliquer une stimulation en au moins plusieurs sites ventriculaires, sur le ventricule gauche, sur le ventricule droit, sur une pluralité de sites à gauche et/ou à droite, etc.

Pour déterminer le(s) site(s) de stimulation optimal(aux), on stimule le ou les ventricules avec les différentes configurations possibles en adaptant les délais entre chaque site de stimulation, et on mesure à chaque fois l'intervalle de temps séparant la stimulation (ou le début de dépolarisation) du ventricule, d'une part, et le début de l'ouverture valvulaire (comme défini ci-dessus), d'autre part, et l'on recherche quelle est la configuration procurant l'intervalle dépolarisation-début d'ouverture le plus court.

Cette dernière valeur est mémorisée comme constituant la valeur optimale de resynchronisation, l'appareil appliquant ensuite, après la phase de réglage, une stimulation contrôlée avec la configuration d'électrodes ainsi sélectionnée.

On pourra ensuite régler, tout en restant sur cette configuration, les autres paramètres de fonctionnement du stimulateur tels que délai atrio-ventriculaire, délai ventricule gauche-ventricule droit, etc.

Selon un autre aspect de l'invention, après la phase de réglage que l'on vient de décrire et qui a permis de sélectionner la configuration optimale, le stimulateur assure un suivi de ce réglage afin de vérifier si l'on se trouve toujours dans la configuration optimale.

Ce suivi est opéré en mesurant, à chaque cycle cardiaque ou à intervalles réguliers, le délai dépolarisation-début d'ouverture et en le comparant à celui qui avait été mémorisé lors de la phase de réglage. Si cet intervalle a augmenté, on peut supposer que la configuration n'est plus optimale et l'on exécute à nouveau une phase de réglage comme décrit précédemment.

On peut ainsi considérer que tout cycle dont l'intervalle en question est supérieur à celui du cycle précédent augmenté d'un pourcentage programmable donné (typiquement d'un pourcentage de 6,25 à 50 %) sera révélateur d'une éventuelle désadaptation de la configuration d'électrodes choisie devant conduire à une nouvelle recherche d'optimisation de la configuration.

On notera que, pour permettre un fonctionnement satisfaisant, on élimine de l'analyse tous les cycles présentant une anomalie, notamment les extrasystoles atriales précoces et les extrasystoles ventriculaires (détection ventriculaire non précédée d'une dépolarisation atriale). On se rapportera avantageusement, pour la définition des extrasystoles ventriculaires, aux FR-A-2 675 695 et EP-A-0 550 342.

Selon un autre aspect de l'invention, le stimulateur discrimine les phases de repos et les phases d'activité du patient, par exemple par une analyse des signaux délivrés par un capteur de ventilation-minute (une telle discrimination entre périodes d'activité et de repos est décrite dans le EP-A-0 719 568). Si, lors d'une phase de repos, l'intervalle temporel entre dépolarisation et début d'éjection n'est pas revenu à la valeur optimale qui avait été mémorisée au cours de la phase de réglage d'initialisation, par exemple après un nombre programmable de cycles sinusaux consécutifs (typiquement un nombre programmable de 1 à 100), la phase de recherche est alors exécutée à nouveau comme décrit plus haut. Si le stimulateur trouve une nouvelle configuration avec un intervalle dépolarisation-début d'éjection plus faible, il sélectionne alors cette configuration et remplace la valeur mémorisée à l'initialisation; dans le cas contraire, il conserve la configuration courante, et se contente de mettre à jour la valeur de l'intervalle dépolarisation-début d'éjection mémorisée comme optimale avec la dernière valeur mesurée au repos.

## Revendications

1. Un stimulateur cardiaque multisites, dans lequel des électrodes sont placées en une pluralité de sites respectifs distincts comprenant au moins deux sites ventriculaires, ces électrodes étant reliées à des bornes indépendantes du stimulateur de manière à permettre la détection d'un potentiel de dépolarisation ainsi que l'application d'une impulsion de stimulation,
**caractérisé par** :
- un capteur de contraction d'au moins l'un des ventricules, pour détecter un instant de début d'ouverture valvulaire ;
- des moyens de recherche de configuration optimale, pour :
. commuter successivement et automatiquement les électrodes ventriculaires selon diverses configurations possibles, et pour détecter un instant de dépolarisation ou de stimulation sur l'électrode dans la configuration correspondante,
. déterminer l'intervalle temporel séparant, pour une configuration donnée, l'instant de dépolarisation ou de stimulation et l'instant de début d'ouverture valvulaire, et
. sélectionner la configuration procurant l'intervalle temporel le plus court ; et
- des moyens pour appliquer une stimulation sur l'électrode dans la configuration sélectionnée par les moyens de recherche.

2. Le stimulateur cardiaque de la revendication 1, comprenant des moyens pour :
- détecter des extrasystoles auriculaires et ventriculaires, et
- inhiber les moyens de recherche en cas de détection d'une extrasystole.

3. Le stimulateur cardiaque de la revendication 1, comprenant en outre des moyens pour :
- comparer la valeur de l'intervalle temporel déterminée pour un cycle cardiaque donné à la valeur correspondante mémorisée pour un cycle antérieur, et
- mettre à nouveau en oeuvre les moyens de recherche en cas d'augmentation de ladite valeur.

4. Le stimulateur cardiaque de la revendication 1, comprenant en outre des moyens pour :
- déterminer un état de repos physiologique du patient et
- mettre à nouveau en oeuvre les moyens de recherche lorsque l'état de repos est détecté.

5. Le stimulateur cardiaque de la revendication 1, dans lequel les moyens de recherche sont mis en oeuvre à intervalles réguliers.

6. Le stimulateur cardiaque de la revendication 1, dans lequel les moyens de recherche sont mis en oeuvre régulièrement après comptage d'un nombre prédéterminé de cycles.

7. Le stimulateur cardiaque de la revendication 1, comprenant en outre des moyens pour adapter les délais entre les sites de stimulation.

## Patentansprüche

1. Multistellen-Herzschrittmacher, in welchem die Elektroden an einer Vielzahl von jeweils verschiedenen Stellen platziert sind, Umfassend mindestens zwei ventrikuläre Stellen, wobei diese Elektroden mit unabhängigen Anschlüssen des Herzschrittmachers verbunden sind, um die Detektion eines Depolarisationspotentials zu erlauben, sowie die Anwendung eines Stimulationsimpulses,
**gekennzeichnet durch**:
- einen Sensor der Kontraktion mindestens eines der Ventrikel, um einen Zeitpunkt des Beginns der Klappenöffnung zu detektieren;
- Mittel zur Suche der optimalen Konfiguration, um:
• aufeinanderfolgend und automatisch die ventrikulären Elektroden gemäß verschiedener möglicher Konfigurationen zu vertauschen, und um einen Zeitpunkt der Depolarisation oder der Stimulation auf der Elektrode in der entsprechenden Konfiguration zu detektieren,
• Bestimmen des Zeitintervalls, welches für eine gegebene Konfiguration, den Zeitpunkt der Depolarisation oder der Stimulation und den Zeitpunkt des Beginns der Öffnung der Klappe trennt, und
• Auswählen der Konfiguration, welche das kürzeste Zeitintervall liefert; und
- Mittel zum Anwenden einer Stimulation auf der Elektrode in der gewählten Konfiguration **durch** die Mittel zur Suche.

2. Herzschrittmacher gemäß Anspruch 1, umfassend Mittel zum:
- Detektieren von atrialen und ventrikulären Extrasystolen, und
- Hemmen der Mittel zur Suche im Fall der Detektion einer Extrasystole.

3. Herzschrittmacher gemäß Anspruch 1, umfassend ferner Mittel zum:
- Vergleichen des Wertes des bestimmten Zeitintervalls für einen gegebenen Herzzyklus mit dem entsprechenden für einen vorhergehenden Zyklus gespeicherten Wert, und
- erneutes in Gang setzen der Mittel zur Suche im Fall der Erhöhung des genannten Werts.

4. Herzschrittmacher gemäß Anspruch 1, umfassend ferner Mittel zum:
- Bestimmen eines physiologischen Riihezustands des Patientens, und
- erneutes in Gang setzen der Mittel zur Suche, wenn der Ruhezustand detektiert wird.

5. Herzschrittmacher gemäß Anspruch 1, in welchem die Mittel zur Suche in regelmäßigen Intervallen in Gang gesetzt werden.

6. Herzschrittmacher gemäß Anspruch 1, in welchem die Mittel zur Suche regelmäßig in Gang gesetzt werden nach Zählen einer vorherbestimmten Anzahl von Zyklen.

7. Herzschrittmacher gemäß Anspruch 1, ferner umfassend Mittel zum Anpassen der Verzögerungen zwischen den Stimulationsstellen.

## Claims

1. A multisite cardiac pacemaker, in which electrodes are placed in a plurality of distinct respective sites comprising at least two ventricular sites, said electrodes being connected to independent terminals of the pacemaker so as to enable the detection of a depolarisation potential as well as the application of a stimulation pulse, **characterised by**:
- a sensor of contraction of at least one of the ventricles, for detecting an time of beginning of valvular opening;
- means for searching an optimal configuration, for:
. successively and automatically switching the ventricular electrodes according to several possible configurations, and for detecting a time of depolarisation or stimulation on the electrode switched in the corresponding configuration,
. determining the time interval separating, for a given configuration, the time of depolarisation or stimulation and the time of valvular opening, and
. selecting the configuration that gives the shortest time interval; and
- means for applying a stimulation on the electrode in the configuration selected by the search means.

2. The cardiac pacemaker of claim 1, comprising means for:
- detecting atrial and ventricular extrasystoles, and
- inhibiting the search means in case of detection of an extrasystole.

3. The cardiac pacemaker of claim 1, further comprising means for:
- comparing the value of the time interval determined for a given cardiac cycle to the corresponding value stored for a prior cycle, and
- further operating the search means in case of increase of said value.

4. The cardiac pacemaker of claim 1, further comprising means for:
- determining a physiological rest state of the patient, and
- further operating the search means when said rest state is detected.

5. The cardiac pacemaker of claim 1, wherein said search means is operated at regular intervals.

6. The cardiac pacemaker of claim 1, wherein said search means is regularly operated after a predetermined number of cycles is counted.

7. The cardiac pacemaker of claim 1, further comprising means to adapt the delays between the stimulation sites.
